# DEMANDE DE BREVET EUROPEEN

(11) **EP 4 818 681 A1**
(43) Date de publication de la demande: **07.10.2026**
(21) Numéro de dépôt: 26167467.5
(22) Date de dépôt: 25.03.2026
(51) Int. Cl.: G01N 21/90, G01N 21/95, G01N 21/88, G01B 11/06

(54) **DISPOSITIF ET PROCÉDÉ D'INSPECTION D'AU MOINS UN RÉCIPIENT**

(30) Priorité: 03.04.2025 FR 2503477
(71) Demandeur: Sidel Participations, 76930 Octeville-sur-Mer (FR)
(72) Inventeur: TONGA, Jonas, 76930 OCTEVILLE-SUR-MER (FR); CAVELIER, Vivien, 76930 OCTEVILLE-SUR-MER (FR)
(74) Mandataire: Sidel Group

(57) **Abrégé**

Le dispositif d'inspection comprend un dispositif de transport (2) du récipient (1) et au moins un module d'inspection (4) du récipient (1) comprenant au moins un dispositif d'acquisition (14) d'un paramètre du récipient.

Le dispositif d'acquisition (14) est mobile selon au moins trois directions :
- en translation selon une direction d'acquisition (D),
- en translation selon une direction d'élévation (Z), et
- en rotation autour d'un axe de rotation sensiblement perpendiculaire à la direction d'acquisition (D) et à la direction d'élévation (Z).

## Description

La présente invention concerne un dispositif d'inspection d'au moins un récipient comprenant un corps s'étendant selon un axe, le dispositif d'inspection étant du type comprenant un dispositif de transport du récipient agencé pour déplacer le récipient selon un trajet de déplacement et au moins un module d'inspection du récipient comprenant un support s'étendant en regard d'une partie du trajet de déplacement selon une direction d'observation sensiblement perpendiculaire à ladite partie du trajet de déplacement et au moins un dispositif d'acquisition d'un paramètre du récipient, ledit dispositif d'acquisition monté sur le support.

L'invention concerne également un procédé d'inspection d'au moins un récipient au moyen d'un tel dispositif d'inspection.

L'invention s'applique par exemple à la mesure de l'épaisseur de la paroi d'une partie d'un récipient à l'issue d'un procédé de réalisation d'un tel récipient, par exemple par étirage soufflage.

Afin de s'assurer qu'une installation de production de récipients produit des récipients présentant les caractéristiques souhaitées et éventuellement évacuer les récipients non conformes, il est connu d'inspecter les récipients à la sortie de l'installation au moyen d'un module d'inspection comprenant au moins un dispositif d'acquisition d'au moins un paramètre du récipient, par exemple l'épaisseur de la paroi d'une partie du récipient, et de déterminer si la valeur du paramètre acquis correspond à la valeur souhaitée pour ce paramètre.

Afin que le paramètre soit correctement acquis, le dispositif d'acquisition doit être placé dans une position précise par rapport au récipient et plus particulièrement par rapport à la partie du récipient inspectée. Dans le cas d'une mesure de l'épaisseur de la paroi, le dispositif d'acquisition doit par exemple être placé sensiblement perpendiculairement à la surface de la paroi et à une distance prédéterminée de celle-ci.

Un tel positionnement précis ne pose généralement pas de problème pour des récipients de section circulaire puisque la surface de la paroi du récipient présente une forme sensiblement uniforme sur tout le corps du récipient.

Cependant, dans le cas d'un récipient de forme plus complexe, par exemple un récipient présentant des arêtes vives, des coins ou autre, le positionnement du dispositif d'acquisition est rendu compliqué lorsqu'il s'agit d'acquérir un paramètre relatif à une zone de forme particulière du corps du récipient.

L'un des buts de l'invention est de pallier cet inconvénient en proposant un dispositif d'inspection permettant d'acquérir simplement et de façon fiable au moins un paramètre du récipient, quelle que soit la forme de celui-ci.

A cet effet, l'invention concerne un dispositif d'inspection du type précité, dans lequel le dispositif d'acquisition est monté de façon mobile sur le support selon au moins trois directions :
- en translation selon une direction d'acquisition pour éloigner ou écarter le dispositif d'acquisition de la partie du trajet de déplacement selon la direction d'acquisition,
- en translation selon une direction d'élévation sensiblement parallèle à l'axe du corps du récipient lorsqu'il est déplacé par le dispositif de transport pour ajuster la hauteur du dispositif d'acquisition par rapport au corps du récipient, et
- en rotation autour d'un axe de rotation sensiblement perpendiculaire à la direction d'acquisition et à la direction d'élévation pour ajuster l'orientation du dispositif d'acquisition dans un plan défini par la direction d'acquisition et par la direction d'élévation.

Les degrés de liberté dans le positionnement du dispositif d'acquisition sur le support permettent d'ajuster de façon fine et précise la position du dispositif d'acquisition par rapport au récipient transporté par le dispositif de transport, quelle que soit la forme du récipient, notamment dans la zone dans laquelle le paramètre doit être acquis. En outre, lorsque des récipients de forme différente doivent être inspectés, le nouveau réglage de la position du dispositif d'acquisition peut se faire rapidement et sans nécessiter d'action manuelle directe sur le module d'inspection.

Le dispositif d'inspection selon l'invention peut comprendre une ou plusieurs des caractéristiques suivantes, prises isolément ou selon toute combinaison techniquement envisageable :
- le support est en outre mobile en rotation autour d'un axe de rotation sensiblement parallèle à l'axe du corps du récipient lorsqu'il est déplacé par le dispositif de transport pour ajuster l'orientation du dispositif d'acquisition en modifiant l'angle formé entre la direction d'observation et la direction d'acquisition ;
- le dispositif d'acquisition est un dispositif de mesure de l'épaisseur d'une paroi d'une partie du corps du récipient ;
- le module d'inspection comprend au moins deux dispositifs d'acquisition montés sur le support, lesdits dispositifs d'acquisition s'étendant à des hauteurs différentes du support selon la direction d'élévation, chaque dispositif d'acquisition étant mobile au moins selon la direction d'acquisition, selon la direction d'élévation et atour d'un axe de rotation ;
- le dispositif d'inspection comprend au moins deux modules d'inspection s'étendant chacun en regard d'une partie du trajet de déplacement, lesdits modules d'inspection étant espacés l'un de l'autre selon le trajet de déplacement ;
- le dispositif de transport est une roue transportant le récipient selon un trajet de déplacement circulaire, le module d'inspection s'étendant en regard d'un secteur angulaire dudit trajet de déplacement circulaire ;
- l'axe de rotation de la roue s'étend selon la direction d'élévation ; et
- le module d'inspection comprend au moins un premier moteur actionnant le déplacement en translation du dispositif d'acquisition selon la direction d'acquisition, un deuxième moteur actionnant le déplacement en translation du dispositif d'acquisition selon la direction d'élévation et un troisième moteur actionnant le déplacement en rotation du dispositif d'acquisition autour de l'axe de rotation.

Selon un autre aspect, l'invention concerne également un procédé d'inspection d'au moins un récipient au moyen d'un dispositif d'inspection tel que décrit ci-dessus, comprenant les étapes suivantes :
- ajuster la position du dispositif d'acquisition selon la direction d'acquisition, selon la direction d'élévation et autour de l'axe de rotation de sorte que le dispositif d'acquisition se trouve dans une position prédéterminée par rapport au corps d'un récipient transporté selon le trajet de déplacement par le dispositif de transport,
- placer le récipient sur le dispositif de transport et déplacer ledit récipient selon le trajet de déplacement, ledit récipient passant en regard du module d'inspection au cours de ce déplacement,
- acquérir au moins un paramètre du récipient au moyen du dispositif d'acquisition lorsque ledit récipient s'étend en regard du dispositif d'acquisition selon la direction d'acquisition.

Selon une caractéristique optionnelle du procédé d'inspection, la direction d'acquisition s'étend perpendiculairement à la surface d'une partie du corps du récipient dans la position prédéterminée, le paramètre du récipient acquis par le dispositif d'acquisition étant relatif à ladite partie du corps du récipient.

D'autres aspects et avantages de l'invention apparaîtront plus clairement à la lecture de la description qui va suivre, donnée uniquement à titre d'exemple non limitatif, et faite en référence aux dessins annexés dans lesquels :
[Fig.1] - la [Fig.1] est une représentation schématique du dessus d'un dispositif d'inspection de récipients selon l'invention,
[Fig.2] - la [Fig.2] est une représentation schématique de côté d'un exemple de récipient pouvant être inspecté au moyen du dispositif d'inspection de la [Fig.1],
[Fig.3] - la [Fig.3] est une représentation schématique en coupe selon une direction d'élévation d'un module d'inspection du dispositif d'inspection de la [Fig.1], et
[Fig.4] - la [Fig.4] est une représentation schématique fonctionnelle du module d'inspection de la [Fig.3].

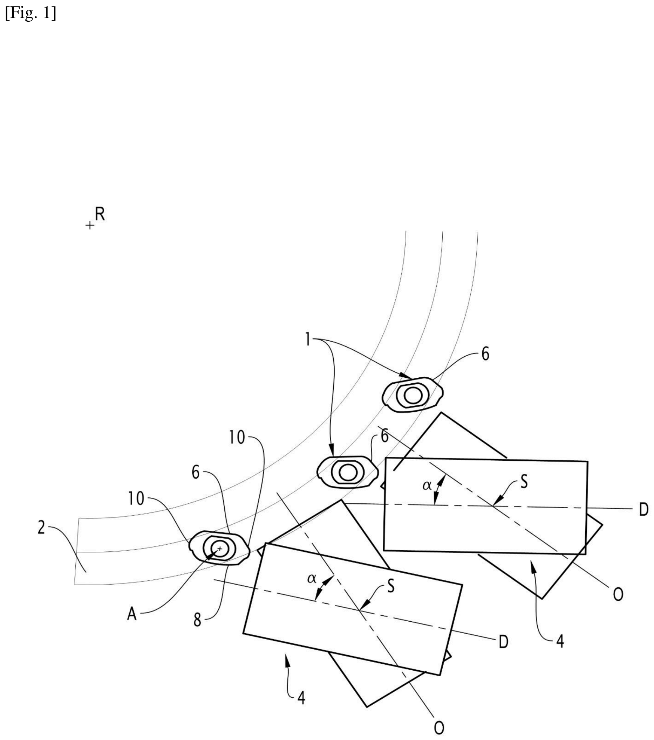

En référence à la [Fig.1], on décrit un dispositif d'inspection d'au moins un récipient 1 comprenant un dispositif de transport 2 du récipient 1 et au moins un module d'inspection 4 du récipient 1. Un tel dispositif d'inspection est par exemple prévu dans une installation de production de récipients afin de vérifier si les récipients produits sont conformes à un modèle de récipient souhaité.

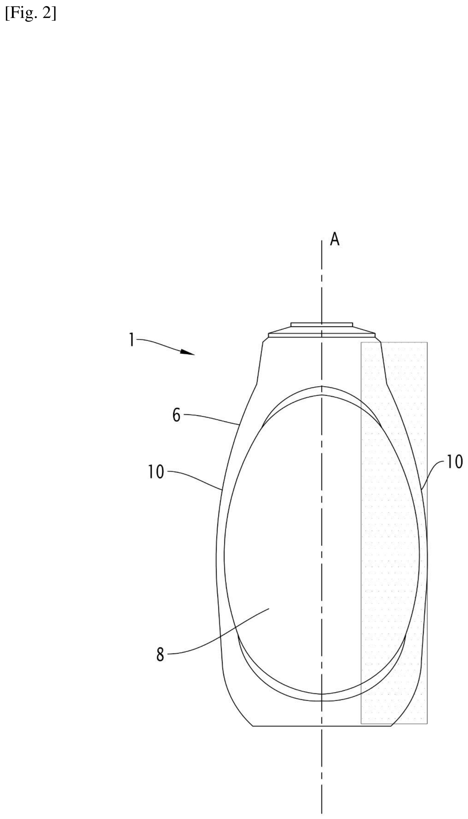

Le récipient 1 comprend un corps 6 s'étendant selon un axe A, comme représenté sur la [Fig.2]. Le récipient 1 est par exemple une bouteille, un flacon, une flasque ou autre. Comme cela sera décrit plus en détail ultérieurement, l'invention est particulièrement avantageuse pour inspecter des récipients présentant une forme « complexe », notamment dont le corps 6 ne présente pas une section circulaire sur toute sa hauteur mesurée selon l'axe A. Ainsi, comme représenté sur les Figs. 1 et 2, le corps 6 du récipient 1 présente par exemple une section de forme sensiblement ellipsoïdale avec deux faces 8 sensiblement planes jointes l'une à l'autre par deux tronçons latéraux, présentant par exemple une section en forme de U, de C ou de V et désignés par le terme tranches 10 dans la suite de la description. Il est entendu que cette forme n'est donnée qu'à titre d'exemple et que l'invention s'applique à des récipients de formes différentes, y compris dont le corps présente une section circulaire.

Le dispositif de transport 2 est agencé pour déplacer le récipient 1 selon un trajet de déplacement. Selon un mode de réalisation, le dispositif de transport 2 est formé par une roue mobile en rotation autour d'un axe de rotation R agencée pour transporter le récipient 1 à sa périphérie selon un trajet de déplacement circulaire. La roue agencée pour maintenir le récipient 1 selon une orientation donnée par rapport à son axe A le long du trajet de déplacement, comme on peut le voir sur la [Fig.1], sur laquelle plusieurs récipients 1 ont été représentés. L'axe de rotation R de la roue est alors sensiblement parallèle à l'axe A du récipient 1 lorsque celui-ci est porté par la roue. Selon un mode de réalisation, le dispositif de transport 2 est agencé pour transporter une succession de récipients 1 les uns à la suite des autres selon le trajet de déplacement. Selon un mode de réalisation, le ou les récipients 1 subissent un traitement particulier lorsqu'ils sont transportés par le dispositif de transport 2, avant ou après être inspectés par le module d'inspection 4. Un tel traitement est par exemple l'application d'un bouchon ou d'une étiquette sur le récipient 1 ou autre.

Le module d'inspection 4 est agencé pour inspecter le ou les récipients 1 alors qu'ils sont transportés selon le trajet de déplacement par le dispositif de transport 2. A cet effet, le module d'inspection 4 est agencé en regard d'une partie du trajet de déplacement selon une direction d'observation O. Par « en regard d'une partie du trajet de déplacement selon une direction d'observation », on entend que le module d'inspection 4 est écarté du trajet de déplacement selon une direction d'observation O qui est sensiblement perpendiculaire à la partie du trajet de déplacement en regard de laquelle s'étend le module d'inspection 4. Dans le cas d'un trajet de déplacement circulaire, la direction d'observation O est ainsi sensiblement perpendiculaire à la tangente de la partie du trajet de déplacement en regard de laquelle s'étend le module d'inspection 4, comme représenté sur la [Fig.1], cette partie du trajet de déplacement étant formée par un secteur angulaire du trajet de déplacement circulaire. Ainsi, le ou chaque récipient 1 passe devant le module d'inspection 4 lorsqu'il est transporté par le dispositif de transport 2. Plus particulièrement, lorsque le trajet de déplacement est circulaire, le ou chaque récipient 1 passe devant le module d'inspection 4 lorsqu'il circule dans le secteur angulaire formant la partie du trajet de déplacement en regard de laquelle s'étend le module d'inspection 4 selon la direction d'observation O.

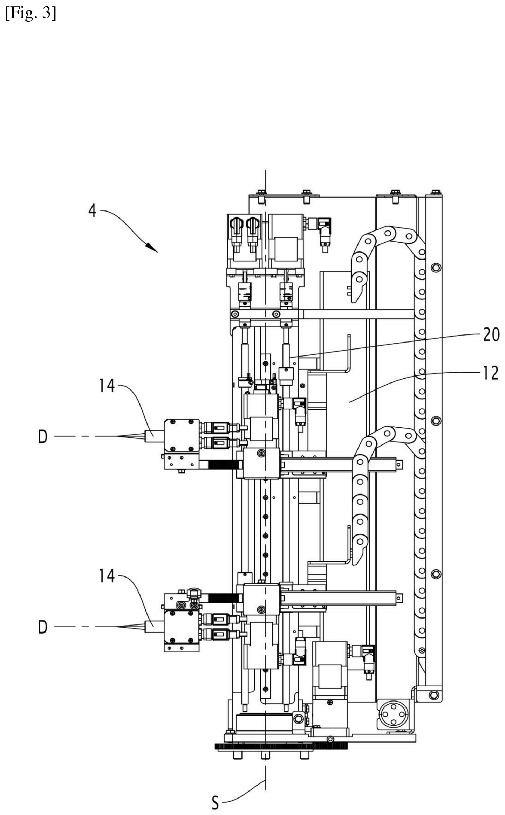

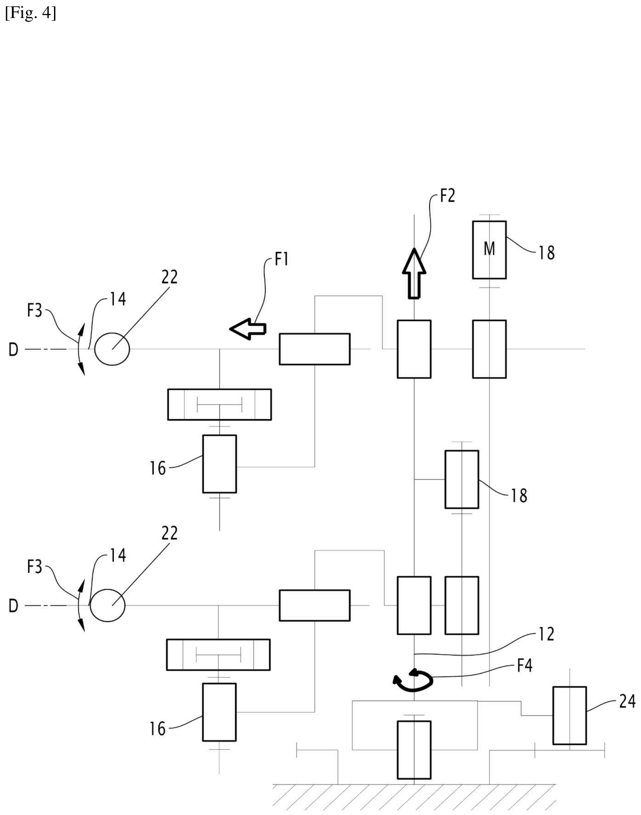

Comme représenté sur les Figs. 3 et 4, le module d'inspection 4 comprend un support 12 et au moins un dispositif d'acquisition 14 monté sur le support 12.

Le support 12 forme une armature adaptée pour recevoir le dispositif d'acquisition 14 ainsi que les moyens permettant le déplacement de celui-ci, comme cela va être décrit ultérieurement.

Le dispositif d'acquisition 14 est agencé pour acquérir au moins un paramètre d'un récipient 1 lorsque ce récipient 1 passe devant le module d'inspection 4 comme décrit précédemment. Le paramètre acquis par le dispositif d'acquisition 14 est par exemple une épaisseur d'une paroi d'une partie du corps 6 du récipient 1. Ainsi, le dispositif d'acquisition 14 est par exemple un dispositif de mesure de l'épaisseur d'une paroi d'une partie du corps 6 du récipient 1 permettant de vérifier si l'épaisseur mesurée est conforme à l'épaisseur souhaitée pour cette partie du corps 6 du récipient et ainsi vérifier si le récipient est conforme au modèle de récipient souhaité et/ou à un cahier des charges. Pour une telle mesure d'épaisseur, il convient de s'assurer que le dispositif d'acquisition 14 acquière l'épaisseur selon une direction d'acquisition D perpendiculaire à la paroi, ou à la surface, de la partie du corps 6 dont on souhaite acquérir l'épaisseur. Une telle exigence peut également s'appliquer à d'autres paramètres du récipient 1.

Ainsi, le dispositif d'acquisition 14 doit être positionné de façon précise par rapport au récipient 1 alors que celui-ci se déplace selon le trajet de déplacement. Ce positionnement n'est pas particulièrement compliqué lorsque la partie du corps 6 du récipient 1 pour laquelle on souhaite acquérir un paramètre présente une section circulaire puisque la forme est constante autour de l'axe A, quelle que soit l'orientation du récipient sur le dispositif de transport 2. Dans ce cas, il suffit que la direction d'acquisition D soit alignée avec la direction d'observation O, puisque la surface du corps 6 du récipient 1 présente une tangente parallèle à celle du trajet de déplacement du récipient.

Par contre, dès lors que le récipient 1 présente une forme complexe, le positionnement précis du dispositif d'acquisition 14 est plus compliqué puisque la forme de la section change autour de l'axe A du corps 6, ce qui change donc la direction d'acquisition D en fonction de la partie du corps 6 pour laquelle on souhaite acquérir le paramètre. Par exemple, si la partie du corps 6 pour laquelle on souhaite acquérir le paramètre se situe sur la tranche 10 d'un récipient 1 tel que décrit précédemment, comme représenté par le rectangle en pointillés sur la [Fig.2], le positionnement du dispositif d'acquisition 14 est particulièrement complexe car la direction perpendiculaire à la paroi change constamment le long de la section de la tranche 10. Ce positionnement peut être d'autant plus compliqué si la tranche 10 présente une forme incurvée, comme représenté sur la [Fig.2], la direction perpendiculaire à la paroi changeant également constamment le long de la hauteur du corps 6.

Afin de permettre un placement précis du dispositif d'acquisition 14 par rapport au corps 6 du récipient 1, le dispositif d'acquisition 14 est monté sur le support 12 de façon mobile selon au moins trois directions permettant de positionner le dispositif d'acquisition 14 dans une orientation souhaitée par rapport à une partie du corps 6 d'un récipient 1 pour laquelle on souhaite acquérir un paramètre, le récipient 1 étant placé sur le dispositif de transport 2 et passant dans la partie du trajet de déplacement en regard de laquelle s'étend le module d'inspection 4.

Selon un premier degré de liberté, le dispositif d'acquisition 14 est mobile en translation par rapport au support 12 selon la direction d'acquisition D, comme représenté par la flèche F₁ de la [Fig.4]. En d'autres termes, le dispositif d'acquisition 14 peut être éloigné ou rapproché du trajet de déplacement selon la direction d'acquisition D, ce qui permet d'adapter la position du dispositif d'acquisition 14 à la dimension radiale du corps 6 du récipient 1 afin que l'extrémité du dispositif d'acquisition 14 s'étende à la distance souhaitée de la surface de la partie du corps 6 pour laquelle on souhaite acquérir un paramètre. Le déplacement du dispositif d'acquisition 14 selon la direction d'acquisition D est par exemple actionné par un premier moteur 16 monté sur le support 12, comme représenté de façon schématique sur la [Fig.4]. Lorsque le premier moteur 16 n'est pas actionné, le dispositif d'acquisition 14 est immobilisé selon la direction d'acquisition D. En d'autres termes, une fois que la position du dispositif d'acquisition 14 a été ajustée selon la direction d'acquisition D, le dispositif d'acquisition 14 est immobile selon la direction d'acquisition D jusqu'à ce qu'un nouveau réglage soit effectué au moyen du premier moteur 16. Ainsi, le déplacement du dispositif d'acquisition 14 selon la direction d'acquisition D n'est pas libre mais contraint à un actionnement du premier moteur 16.

Selon un deuxième degré liberté, le dispositif d'acquisition 14 est mobile en translation par rapport au support 12 selon la direction d'élévation Z, comme représenté par la flèche F₂ de la [Fig.4]. En d'autres termes, la hauteur du dispositif d'acquisition 14 sur le support 12 peut être ajustée le long de l'axe A du corps 6 du récipient 1 lorsque celui-ci est transporté par le dispositif de transport 2 en fonction de la hauteur de la partie du corps 6, mesurée selon l'axe A, pour laquelle on souhaite acquérir un paramètre. Le déplacement du dispositif d'acquisition 14 selon la direction d'élévation Z est par exemple actionné par un deuxième moteur 18 monté sur le support 12, comme représenté de façon schématique sur la [Fig.4]. Lorsque le deuxième moteur 18 n'est pas actionné, le dispositif d'acquisition 14 est immobilisé selon la direction d'élévation Z. En d'autres termes, une fois que la position du dispositif d'acquisition 14 a été ajustée selon la direction d'élévation Z, le dispositif d'acquisition 14 est immobile selon la direction d'élévation Z jusqu'à ce qu'un nouveau réglage soit effectué au moyen du deuxième moteur 18. Ainsi, le déplacement du dispositif d'acquisition 14 selon la direction d'élévation Z n'est pas libre mais contraint à un actionnement du deuxième moteur 18. Comme représenté sur la [Fig.3], le module d'inspection 4 comprend par exemple un élément de transmission 20 entre le deuxième moteur 18 et le dispositif d'acquisition 14, permettant de déplacer le dispositif d'acquisition 14 selon la direction d'élévation Z lorsque le deuxième moteur 18 est actionné. Un tel élément de transmission 20 est par exemple formé par une vis à bille sur la [Fig.3].

Selon un troisième degré de liberté, le dispositif d'acquisition 14 est mobile en rotation par rapport au support 12 autour d'un axe de rotation sensiblement perpendiculaire à la direction d'acquisition et à la direction d'élévation, comme représenté par une flèche F₃ de la [Fig.4]. En d'autres termes, l'orientation du dispositif d'acquisition 14 peut être ajustée dans un plan contenant la direction d'acquisition D et la direction d'élévation Z afin de modifier la direction d'acquisition D dans ce plan. Une telle orientation permet de faire pointer le dispositif d'acquisition vers le haut ou vers le bas afin d'adapter la direction d'acquisition D à la forme de la partie du corps 6 du récipient 1 pour laquelle on souhaite acquérir un paramètre. Une telle orientation permet notamment de tenir compte de l'incurvation du corps 6, par exemple au niveau de la tranche 10 comme représenté sur la [Fig.2], afin d'assurer que la direction d'acquisition soit perpendiculaire à la partie du corps 6 souhaitée y compris lorsque celle-ci ne s'étend pas dans un plan parallèle à l'axe A du corps 6. Le déplacement du dispositif d'acquisition 14 autour de son axe de rotation est par exemple actionné par un troisième moteur 22 monté sur le support 12, comme représenté de façon schématique sur la [Fig.4]. Lorsque le troisième moteur 22 n'est pas actionné, le dispositif d'acquisition 14 est immobilisé autour de son axe de rotation. En d'autres termes, une fois que la position du dispositif d'acquisition 14 a été ajustée autour de son axe de rotation, le dispositif d'acquisition 14 est immobile autour de cet axe jusqu'à ce qu'un nouveau réglage soit effectué au moyen du troisième moteur 22. Ainsi, le déplacement du dispositif d'acquisition 14 autour de son axe de rotation n'est pas libre mais contraint à un actionnement du troisième moteur 22.

Selon un mode de réalisation, lorsque les récipients 1 à inspecter sont déplacés par le dispositif de transport 2 de telle sorte qu'au moins une partie pour laquelle on veut acquérir un paramètre ne s'étend pas parallèlement à la tangente au trajet de circulation dans le secteur angulaire en regard du module d'inspection 4, l'orientation de la direction d'acquisition D par rapport à la direction d'observation O est également ajustable, comme représenté sur la [Fig. 1]. A cet effet, selon ce mode de réalisation, le support 12 du module d'inspection 4 est mobile en rotation autour d'un axe de rotation S sensiblement parallèle à l'axe A du corps 6 du récipient 1 lorsqu'il est déplacé par le dispositif de transport 2 pour ajuster l'orientation du dispositif d'acquisition 14 en modifiant l'angle α formé entre la direction d'observation O et la direction d'acquisition D, comme représenté sur la [Fig.1] et comme représenté par la flèche F₄ de la [Fig.4]. Sur cette figure, la position du module d'inspection 4 lorsque la direction d'acquisition D est confondue avec la direction d'observation O est représentée en pointillés et la position dans laquelle la direction d'acquisition D forme un angle α non nul avec la direction d'observation O est représentée en traits pleins. Le déplacement du support 12 autour de son axe de rotation S est par exemple actionné par un quatrième moteur 24, comme représenté de façon schématique sur la [Fig.4]. Lorsque le quatrième moteur 24 n'est pas actionné, le support 12 est immobilisé autour de son axe de rotation S. En d'autres termes, une fois que la position du support 12 a été ajustée autour de son axe de rotation S, le support 12 est immobile autour de cet axe jusqu'à ce qu'un nouveau réglage soit effectué au moyen du quatrième moteur 24. Ainsi, le déplacement du support 12 autour de son axe de rotation S n'est pas libre mais contraint à un actionnement du quatrième moteur 24.

Les ajustements possibles du module d'inspection 4 décrits ci-dessus permettent ainsi de position de façon précise le dispositif d'acquisition 14 pour que le paramètre acquis par celui-ci le soit de façon optimale en orientant la direction d'acquisition D pour qu'elle soit strictement perpendiculaire à la surface de la partie du corps 6 du récipient 1 pour laquelle on souhaite acquérir le récipient 1. Ces ajustements sont faits pour un récipient 1, par exemple placé en regard du module d'inspection 4 sur le dispositif de transport 2 sans que celui-ci ne déplace le récipient 1. Une fois que ces ajustements ont été faits par l'actionnement des différents moteurs, le dispositif d'acquisition 14 se trouve dans une position prédéterminée fixe et l'inspection d'un ou plusieurs récipients 1 peut débuter. Pour ce faire, une succession de récipients 1 est placée sur le dispositif de transport 2 et défile devant le module d'inspection 4 avec le dispositif d'acquisition 14 dans la position prédéterminée. Celui-ci acquière alors le paramètre souhaité pour chaque récipient 1. Cette acquisition se fait pour la même partie du corps 6 de tous les récipients de sorte qu'on assure une répétabilité de la mesure du paramètre souhaité alors que ceux-ci se déplacent à vitesse élevée selon le trajet de déplacement. Une telle inspection des récipients 1 permet de s'assurer que les récipients 1 sont conformes à un modèle de récipient souhaité et/ou à un cahier des charges et une mise à l'écart peut être prévue pour le ou les récipients dont le paramètre acquis ne correspond pas à un paramètre attendu ou n'est pas compris dans une plage de valeurs acceptable pour ce paramètre.

Lorsque le type de récipients à inspecter est modifié, le réglage de la position du dispositif d'acquisition 14 peut être à nouveau effectué, de façon rapide grâce aux différents moteurs du module d'inspection 4 et sans qu'un opérateur ait besoin d'agir manuellement directement sur le module d'inspection 4 pour déplacer puis immobiliser le dispositif d'acquisition 14 sur le support 12.

Selon le mode de réalisation représenté sur les Figs. 3 et 4, le module d'inspection 4 comprend au moins deux dispositifs d'acquisition 14 tels que décrits précédemment. Ces dispositifs d'acquisition 14 sont montés sur le support 12 à des hauteurs différentes du support 12 selon la direction d'élévation Z. Chaque dispositif d'acquisition 14 est alors mobile au moins selon la direction d'acquisition D, selon la direction d'élévation Z et atour d'un axe de rotation, comme décrit précédemment, par exemple au moyen de moteurs dédiés pour chaque dispositif d'acquisition 14. L'orientation de la direction d'acquisition D de chaque dispositif d'acquisition 14 est de préférence ajustable indépendamment de celle de la direction d'acquisition du ou des autres dispositifs d'acquisition 14 afin de tenir compte des variations éventuelles de la forme du corps 6 du récipient 1 le long de son axe A. Les dispositifs d'acquisition 14 du module d'inspection 4 peuvent être prévus pour acquérir le même paramètre en des endroits différents du corps 6 ou des paramètres différents.

Selon le mode de réalisation représenté sur la [Fig. 1], le dispositif d'inspection comprend au moins deux modules d'inspection 4, comprenant chacun un support 12 sur lequel sont montés un ou plusieurs dispositifs d'acquisition 14. Les modules d'inspection 4 sont espacés l'un de l'autre selon le trajet de déplacement des récipients de sorte à être placés en regard de parties différentes de ce trajet de déplacement. Un tel mode de réalisation permet notamment d'inspecter une même région d'un récipient 1 en tenant compte du fait que ce récipient 1 peut tourner autour de son axe A alors qu'il est transporté par le dispositif de transport 2. A cet effet, en plus des déplacements possibles de chaque dispositif d'acquisition 14, le support 12 de chaque module d'acquisition 4 est par exemple mobile en rotation autour de son axe de rotation S respectif. De préférence, le réglage de la position du support 12 d'un module d'inspection 4 autour de son axe de rotation S peut se faire indépendamment de ce réglage pour le ou les autres modules d'inspection 4. Ainsi, les modules d'inspection 4 peuvent être utilisés pour acquérir un ou plusieurs paramètres pour une ou plusieurs mêmes parties du corps 6 de récipients 1 ou pour des parties différentes du corps 6 de récipients 1 en tenant compte d'un changement d'orientation des récipients 1 alors qu'ils se déplacent selon le trajet de déplacement.

## Revendications

1. Dispositif d'inspection d'au moins un récipient (1) comprenant un corps (6) s'étendant selon un axe (A), le dispositif d'inspection comprenant un dispositif de transport (2) du récipient (1) agencé pour déplacer le récipient (1) selon un trajet de déplacement et au moins un module d'inspection (4) du récipient (1) comprenant un support (12) s'étendant en regard d'une partie du trajet de déplacement selon une direction d'observation (O) sensiblement perpendiculaire à ladite partie du trajet de déplacement et au moins un dispositif d'acquisition (14) d'un paramètre du récipient, ledit dispositif d'acquisition (14) monté sur le support (12), **caractérisé en ce que** le dispositif d'acquisition (14) est monté de façon mobile sur le support (12) selon au moins trois directions :
- en translation selon une direction d'acquisition (D) pour éloigner ou écarter le dispositif d'acquisition (14) de la partie du trajet de déplacement selon la direction d'acquisition (D),
- en translation selon une direction d'élévation (Z) sensiblement parallèle à l'axe (A) du corps (6) du récipient (1) lorsqu'il est déplacé par le dispositif de transport (2) pour ajuster la hauteur du dispositif d'acquisition (14) par rapport au corps (6) du récipient (1), et
- en rotation autour d'un axe de rotation sensiblement perpendiculaire à la direction d'acquisition (D) et à la direction d'élévation (Z) pour ajuster l'orientation du dispositif d'acquisition (14) dans un plan défini par la direction d'acquisition (D) et par la direction d'élévation (Z).

2. Dispositif d'inspection selon la revendication 1, dans lequel le support (12) est en outre mobile en rotation autour d'un axe de rotation (S) sensiblement parallèle à l'axe (A) du corps (6) du récipient (1) lorsqu'il est déplacé par le dispositif de transport (2) pour ajuster l'orientation du dispositif d'acquisition (14) en modifiant l'angle (α) formé entre la direction d'observation (O) et la direction d'acquisition (D).

3. Dispositif d'inspection selon la revendication 1 ou 2, dans lequel le dispositif d'acquisition (14) est un dispositif de mesure de l'épaisseur d'une paroi d'une partie du corps (6) du récipient (1).

4. Dispositif d'inspection selon l'une quelconque des revendications 1 à 3, dans lequel le module d'inspection (4) comprend au moins deux dispositifs d'acquisition (14) montés sur le support (12), lesdits dispositifs d'acquisition (14) s'étendant à des hauteurs différentes du support (12) selon la direction d'élévation (Z), chaque dispositif d'acquisition (14) étant mobile au moins selon la direction d'acquisition (D), selon la direction d'élévation (Z) et atour d'un axe de rotation.

5. Dispositif d'inspection selon l'une quelconque des revendications 1 à 4, comprenant au moins deux modules d'inspection (4) s'étendant chacun en regard d'une partie du trajet de déplacement, lesdits modules d'inspection (4) étant espacés l'un de l'autre selon le trajet de déplacement.

6. Dispositif d'inspection selon l'une quelconque des revendications 1 à 5, dans lequel le dispositif de transport (2) est une roue transportant le récipient (1) selon un trajet de déplacement circulaire, le module d'inspection (4) s'étendant en regard d'un secteur angulaire dudit trajet de déplacement circulaire.

7. Dispositif d'inspection selon la revendication 6, dans lequel l'axe de rotation (R) de la roue s'étend selon la direction d'élévation (Z).

8. Dispositif d'inspection selon l'une quelconque des revendications 1 à 7, dans lequel le module d'inspection (4) comprend au moins un premier moteur (16) actionnant le déplacement en translation du dispositif d'acquisition (14) selon la direction d'acquisition (D), un deuxième moteur (18) actionnant le déplacement en translation du dispositif d'acquisition (14) selon la direction d'élévation (Z) et un troisième moteur (22) actionnant le déplacement en rotation du dispositif d'acquisition (14) autour de l'axe de rotation.

9. Procédé d'inspection d'au moins un récipient au moyen d'un dispositif d'inspection selon l'une quelconque des revendications 1 à 8, comprenant les étapes suivantes :
- ajuster la position du dispositif d'acquisition (14) selon la direction d'acquisition (D), selon la direction d'élévation (Z) et autour de l'axe de rotation de sorte que le dispositif d'acquisition (14) se trouve dans une position prédéterminée par rapport au corps (6) d'un récipient (1) transporté selon le trajet de déplacement par le dispositif de transport (2),
- placer le récipient (1) sur le dispositif de transport (2) et déplacer ledit récipient (1) selon le trajet de déplacement, ledit récipient (1) passant en regard du module d'inspection (4) au cours de ce déplacement,
- acquérir au moins un paramètre du récipient (1) au moyen du dispositif d'acquisition (14) lorsque ledit récipient (1) s'étend en regard du dispositif d'acquisition (14) selon la direction d'acquisition (D).

10. Procédé d'inspection selon la revendication 9, dans lequel la direction d'acquisition (D) s'étend perpendiculairement à la surface d'une partie du corps (6) du récipient (1) dans la position prédéterminée, le paramètre du récipient acquis par le dispositif d'acquisition (14) étant relatif à ladite partie du corps (6) du récipient (1).
